# EUROPEAN PATENT SPECIFICATION

(11) **EP 2 645 965 B1**
(45) Date of publication and mention of the grant of the patent: **10.08.2016**
(21) Application number: 11845811.6
(22) Date of filing: 21.11.2011
(51) Int. Cl.: A61F 2/44

(54) **LATERAL SPONDYLOLISTHESIS REDUCTION CAGE**
KÄFIG ZUR LINDERUNG SEITLICHER SPONDYLOLISTHESE
CAGE DE RÉDUCTION DE SPONDYLOLISTHÉSIS LATÉRAL

(30) Priority: 17.06.2011 US 201113163427; 30.11.2010 US 397716 P
(43) Date of publication of application: 09.10.2013
(73) Proprietor: DePuy Synthes Products, LLC, Raynham, MA 02767-0350 (US)
(72) Inventor: O'NEIL, Michael, J., Raynham, MA 02767 (US); SERHAN, Hassan, Raynham, MA 02767 (US); RAYMOND, Douglas, Raynham, MA 02767 (US); HAWKINS, John, Riley, Raynham, MA 02767 (US)
(74) Representative: Orr, Robert
(86) International application number: PCT/US2011/061611
(87) International publication number: WO 2012/074809

(56) References cited:
- WO-A1-99/63914
- DE-A1- 10 357 960
- FR-A1- 2 874 814
- US-A- 6 126 689
- US-A1- 2003 135 275
- US-A1- 2003 191 531
- US-A1- 2005 177 173
- US-A1- 2006 058 807
- US-A1- 2008 082 173
- US-A1- 2008 234 733
- US-A1- 2010 016 968
- US-A1- 2010 249 935
- US-A1- 2010 280 619

## Description

### BACKGROUND OF THE INVENTION

Lateral interbody fusion procedures are currently indicated for patients with ≤ grade 1 spondylolisthesis. However, correction from the lateral approach is currently limited to regaining height and lordosis with only a low degree of spondylolisthesis correction, as the straight or lordotic cage is impacted during insertion to expand the disc space. Significant spondylolisthesis reduction is currently accomplished via a posterior approach with supplemental posterior fixation devices, including facet screws, translaminar screws, pedicle screws and rods, as well as intraspinous process devices or plates.

Although current lateral cages are characterized by symmetric superior and inferior geometries, the normal and degenerated discs do not have such similar superior and inferior endplate geometries. The lack of conformity of the cage endplate to the pertinent vertebral body can promote cage malpositioning during insertion, improper load balancing, increased settling and/or subsidence, as well as device movement following implantation.

Some surgeons using lateral cages attach lateral plating to the cage to achieve enhanced cage securement accompanied by some degree of biomechanical stabilization. However, most currently available lateral cages do not provide for plate attachment.

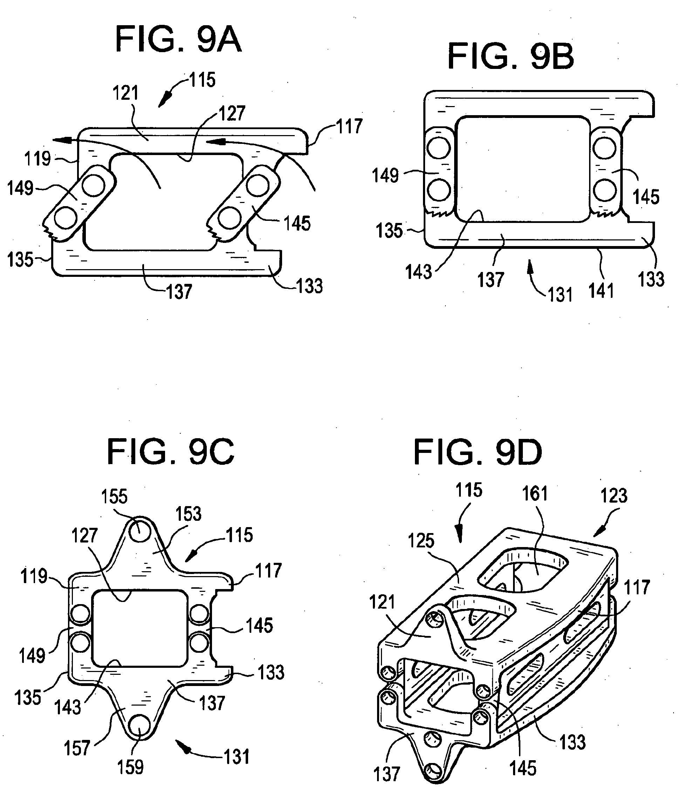

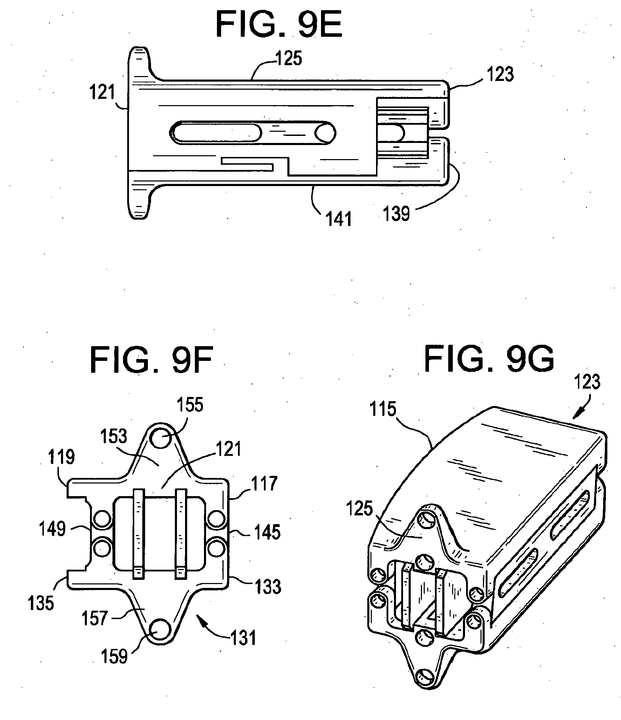

US 2004-0220668 (Eisermann) discloses a method for correcting spondylolisthesis from the lateral approach is provided in which a pair of insertion members are inserted laterally into upper and lower vertebrae, a connecting member is affixed to the insertion members, and a rotating force is applied to the connecting member to encourage the upper and lower vertebrae into a desired position relative to one another. In FIGS. 9-11 of Eisermann, in an alternative embodiment, a slidable prosthetic joint can be used to help with the lateral approach for treating spondylolisthesis. The sliding joint extends generally along the longitudinal axis and includes a first slidable component and a second slidable component. The slidable components cooperate to form the sliding joint which is sized and configured for disposition within an intervertebral space between adjacent vertebral bodies. The sliding joint provides movement between the adjacent vertebral bodies to maintain or restore some of the motion similar to the normal bio-mechanical motion provided by a natural intervertebral disc. More specifically, the slidable components are permitted to translate relative to one another in the axial plane.

US Patent Publication No. 2010-0016968 (Moore) discloses an apparatus and method that allow for the realignment and stabilization of adjacent vertebrae. An implant of this invention both repositions adjacent vertebrae and remains in situ to maintain the new position. The implant has two halves which are interlocked such that they can slide horizontally with respect to each other. Movement of the implant halves and their respective positions are controlled by external set screw and internal locking block within the implant. The implant includes radial anchors which fit into alignment slots made in the misaligned vertebra by the disclosed method. The set screws are used to advance the halves of the implant which in turn move the misaligned vertebrae back into correct positions. The correct position of the vertebrae is locked in place through a bolt and a plate.

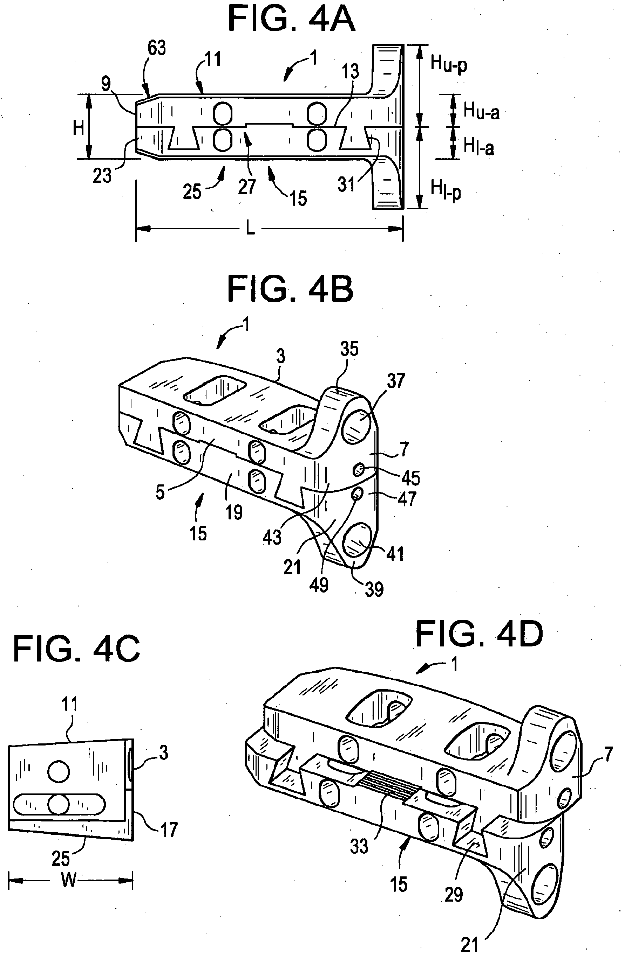

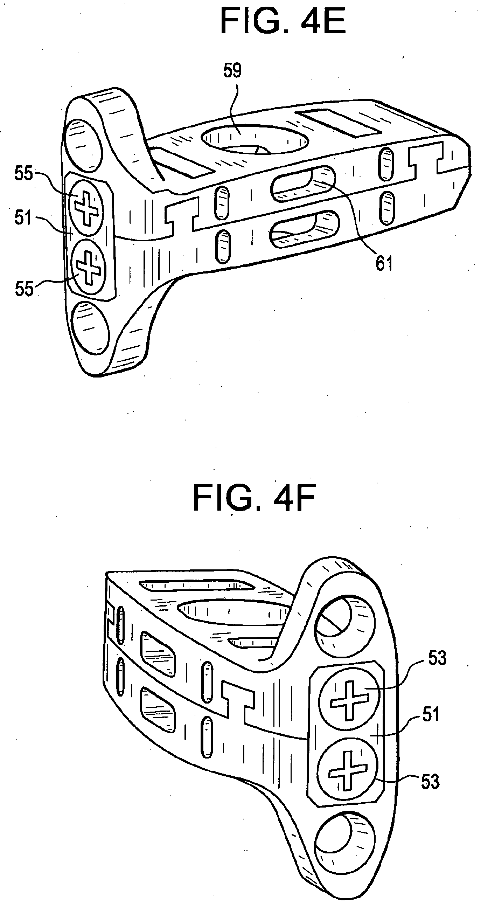

US Patent No. 6342074 (Simpson) discloses a spinal fusion implant and method for maintaining proper lumbar spine curvature and intervertebral disc spacing where a degenerative disc has been removed. The one-piece implant comprises a hollow body having an access passage for insertion of bone graft material into the intervertebral space after the implant has been affixed to adjacent vertebrae. The implant provides a pair of screw-receiving passages that are oppositely inclined relative to a central plane. In one embodiment, the screw-receiving passages enable the head of an orthopedic screw to be retained entirely within the access passage. A spinal fusion implant embodied in the present invention may be inserted anteriorally or laterally. FIG. 4 discloses a device having fixtures for attaching to a lateral side of a vertebral body.

US Patent No. 6,878,167 (Ferree) discloses an osteotomy of a portion of a vertebral endplate and/or vertebral body allowing for easier insertion of a device that fits tightly into a disc space. It also discloses a mechanical device to hold the osteotomized portion of the vertebra against the vertebral body after the intradiscal device is placed. The device may be removed after the pieces of vertebra heal and fuse together. It further discloses a device secured to a side of the vertebral body in FIG. 4C.

US Patent Publication No. 2003/0135275 (Garcia) discloses a spinal implant for establishing a separation between adjacent vertebrae. The implant comprises two or more members which are joined together to form the implant, via a rotational dovetail joint. The rotational dovetail joint inhibits separation and axial movement of the members relative to each other. A fastener is press-fitted into an opening that extends through a first member and into an opening in a second member, to inhibit rotation or the first member relative to the second member. Serrations on the members forming the implant inhibit backout of the implant after insertion.

### SUMMARY OF THE INVENTION

The present invention provides an intervertebral fusion device as claimed in claim 1.

Optional further features of the intervertebral fusion device are set out in the dependent claims.

The present invention relates to an intervertebral fusion device which may comprise inferior and superior fusion cages that provide an ability to correct spondylolisthesis via lateral insertion and *in-situ* adjustment*.*

The intervertebral fusion device for correcting spondylolisthesis in a patient of the present invention comprises:
a) an upper cage having an anterior wall, a posterior wall, and a proximal wall and a distal wall connecting the anterior and posterior walls, and an upper surface for contacting an upper vertebral body and a lower surface,
b) a lower cage having an anterior wall, a posterior wall, and a proximal wall and a distal wall connecting the anterior and posterior walls, and a lower surface for contacting a lower vertebral body and an upper surface,
wherein the lower surface of the upper cage slidingly mates with the upper surface of the lower cage.

A method for correcting spondylolisthesis in a patient is also disclosed, comprising the steps of:
a) selecting a fusion device comprising an upper cage and a lower cage,
b) fixing the upper cage to an upper vertebral body of the patient and the lower cage to a lower vertebral body of the patient, and
c) moving the upper cage relative to the lower cage to correct the spondylolisthesis.

Another intervertebral fusion device for correcting spondylolisthesis in a patient is disclosed, which does not fall within the scope of the invention as claimed, the device comprising:
a) an upper cage having an anterior wall, a posterior wall, and a proximale wall and a distal wall connecting the anterior and posterior walls, and an upper surface for contacting an upper vertebral body and a lower surface having a first groove therein,
b) a lower cage having an anterior wall, a posterior wall, and a proximal wall and a distal wall connecting the anterior and posterior walls, and a lower surface for contacting a lower vertebral body and an upper surface having a second groove therein, and
c) a locking plate,
wherein the lower surface of the upper cage contacts the upper surface of the lower cage so that the first and second grooves form a first throughhole running from the proximal wall to about the distal wall.
wherein the locking plate is disposed in the first throughole.

Another intervertebral fusion device for correcting spondylolisthesis in a patient is disclosed, which does not fall within the scope of the invention as claimed, the device comprising:
a) an upper cage having an anterior wall, a posterior wall, and a proximal wall and a distal wall connecting the anterior and posterior walls, and an upper surface for contacting an upper vertebral body and a lower surface,
b) a lower cage having an anterior wall, a posterior wall, and a proximal wall and a distal wall connecting the anterior and posterior walls, and a lower surface for contacting a lower vertebral body and an upper surface,
wherein the anterior wall is connected to the proximal wall by a first dual linkage and to the distal wall by a second dual linkage,
wherein the posterior wall is connected to the proximal wall by a third dual linkage and to the distal wall by a fourth dual linkage.
wherein the linkages allow the upper plate to pivot relative to the lower plate in the plane of the proximal wall.

Another intervertebral fusion device for correcting spondylolisthesis in a patient is disclosed, which does not fall within the scope of the invention as claimed, the device comprising:
a) an upper wall having an upper surface adapted for contacting an upper vertebral body and an inner surface,
b) a lower wall having a lower surface adapted for contacting a lower vertebral body and an inner surface,
c) proximal and distal walls extending between the upper and lower walls,
d) anterior and posterior walls extending between the upper and lower walls,
e) a rack-and-pinion mechanism located between the inner surfaces of the upper and lower walls
wherein the pinion extends substantially from the distal wall to the proximal wall,
wherein the rack extends substantially from the anterior wall to the posterior wall,
so that rotation of the pinion effects relative movement of the upper and lower walls in the anterior-posterior direction.

### DESCRIPTION OF THE DRAWINGS

FIG.1 is a side view of a functional spinal unit having grade 1 spondylolisthesis characterized by a 20-25% slip.
FIGS 2a-2b and 3 disclose the implantation of the fusion device of the present invention, in which the cages of the device are implanted into the disc space, brought together, and then locked in place.
FIGS. 4a-4d show various views of the intervertebral fusion device of the present invention.
FIGS. 4e-4f show various views of the cages of an intervertebral fusion device of the present invention locked with a locking plate.
FIGS 5a-b disclose a compression-directed inserter having distal pins, and the insertion of a cage of the present invention with this inserter..
FIGS 5c-d disclose a compression-directed inserter having distal blades, and the insertion of a cage of the present invention with this inserter..
FIGS. 6a-7b disclose how a compression-directed inserter
having nested blades aligns the cages of the present invention when activated.
FIGS. 8a-8d disclose how the cages of one embodiment of the present invention are aligned by a rotary spreader, and are locked by a particular locking plate.
FIGS. 9a-9g disclose various views of a dual linkage embodiment of the present invention.
FIGS. 10a-d disclose full and partial views of a rack-and-pinion embodiment of the present invention, some of which are inserted into a disc space.
FIGS 11a-11e disclose various views of the rack-and-pinion embodiment of the present invention.
FIG. 12 discloses a lateral cage of the present invention with oblique screwholes.
FIG. 13 discloses an inserter.

### DETAILED DESCRIPTION OF THE INVENTION

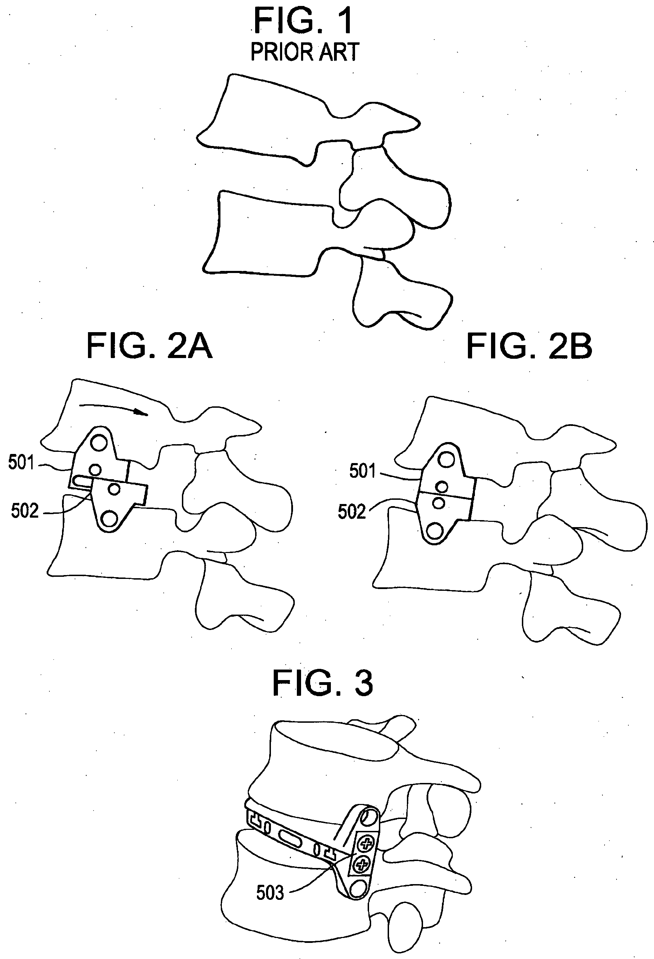

FIG.1 is a side view of a functional spinal unit having grade 1 spondylolisthesis characterized by a 20-25% slip.

In use, the devices of the present invention accomplish improved and controlled spondylolisthesis correction with fusion from the lateral approach. The lateral cage devices of the present invention also provide for intra-operative trialing and selection to enhance conformance of the cage geometry to the vertebral body endplates bounding the targeted disc space. The fusion device of the present invention provides for direct attachment of its superior and inferior cages to the lateral aspects of the opposing vertebral bodies.

Several devices and methods for correcting spondylolisthesis with fusion from the lateral approach are disclosed. All incorporate a superior and inferior fusion cages that are fixedly attached to the corresponding vertebral bodies. The fixed attachment can be accomplished by using pre-attached plates, or by incorporating internal screws (e.g., the STALIF approach) and/or lateral keels. Following implantation, the superior and inferior cages are aligned *in-situ* via various activation means that are further discussed below, and then locked in place.

The interior, contacting surfaces of the cages that effect intra-device attachment contain alignment and securement features that allow for controlled intra-operative manipulation of the spine in the sagittal plane following individual fixed attachment of the cages to the superior and inferior vertebral bodies. These features can include but are not limited to teeth, barbs, and dovetails.

Both the superior and inferior cages can include features on their outer surfaces that can enhance securement to the vertebral body endplate. These features include fins, barbs, teeth, osteoconductive surface morphology (porosity) and coatings (such as HA and TCP). The superior and inferior cages can also include graft-retention windows and pockets to facilitate the long-term fusion of the two vertebral bodies of the functional spinal unit.

The inner contacting surfaces of the cage can be flat to allow for the incremental lineal adjustment of the relative cage positions. Alternatively, these surfaces can be domed so as to enable the accurate adjustment of the vertebral bodies to a centered position in the flexion/extension plane (i.e., to the center of rotation).

The external geometry of the superior and inferior cages can be flat or lordotic, and can be domed or angled in various plans to enhance their conformance to the vertebral body endplates and to address spinal deformity and/or scoliosis.

Following fixed attachment to the vertebral body, the superior and inferior cages may be aligned by several means, including the following:

FIGS 2a-2b and 3 disclose the implantation of a first embodiment of a fusion device of the present invention, in which the cages 501,502 of the device are implanted into the disc space, brought together, and then locked in place with a locking plate 503. FIGS. 4a-4d show various views of the intervertebral fusion device of the present invention. FIGS. 4e-4f show various views of the cages of an intervertebral fusion device of the present invention locked with a locking plate.

FIG. 2a and FIG. 2b respectively show the relative positions of the fixed cage before and after alignment. In the FIG. 2b position, the cages have properly aligned the vertebral bodies, and thereby corrected the spondylolisthesis. The superior and inferior cages can also have features that provide or enhance the connection of the cages to the compressor. These features include recesses, pilot holes and threads located on the proximal walls of the two cages (and optionally extending therethrough) that receive mating features located on the cage inserter. These features may also assist in the alignment of the cages. Lastly, the upper portion of the proximal wall of the upper cage and the lower portion of the proximal wall of the lower cage each extend past the anterior and posterior walls of the respective cages, and each has a transverse throughhole. Fixation screws may extend through these holes and into the lateral walls of the corresponding vertebral bodies to provide the immediate fixation of the cages to these vertebral bodies. Such screw locking features are well known in the art.

As shown in FIGS. 4a,4b and 4d, the superior and inferior cages together preferably form a dovetail joint (or other joint having an expanding recess) that allows linear anterior-posterior relative movement of the fixed cages to effect the desired alignment of the vertebral bodies. The contacting surfaces of the superior and inferior cages may also have matching ratchet teeth, as in FIG. 4d, that provide incremental adjustment of the relative cage positions, and the short term inter-cage securement following the compression.

As shown in FIGS. 4e and f, once the superior and inferior cages are aligned together, this desired position may be fixed by attaching a single locking plate to the proximal ends of each cage. This locking plate may be attached to the cages by passing screws through the holes in the plate and into the corresponding holes in the lower portion of the proximal wall of the upper plate and the upper portion of the proximal wall of the lower plate.

Now referring to FIGS. 4a-4f, there is provided (claim 1) an intervertebral fusion device for correcting spondylolisthesis in a patient, comprising:
a) an upper cage 1 having an anterior wall 3, a posterior wall 5, and a proximal wall 7 and a distal wall 9 connecting the anterior and posterior walls, and an upper surface 11 for contacting an upper vertebral body and a lower surface 13,
b) a lower cage 15 having an anterior wall 17 , a posterior wall 19 , and a proximal wall 21 and a distal wall 23 connecting the anterior and posterior walls, and a lower surface 25 for contacting a lower vertebral body and an upper surface 27,
wherein the lower surface of the upper cage slidingly mates with upper surface of the lower cage.

In some embodiments, the lower surface of the upper plate and the upper surface of the lower cage include tongue-and- groove feature. Preferably, the tongue-and-groove feature runs from about the anterior wall to about the posterior wall. Preferably, the tongue-and-groove feature comprises an expanding recess 29 and more preferably comprises a dovetail 31.

In some embodiments, the lower surface of the upper cage and the upper surface of the lower cage include a ridge and recess feature 33 that runs in a proximal -distal direction.

In some embodiments, the proximal wall of the upper cage has a height Hu-p, the anterior wall of the upper cage has a height Hu-a, and wherein the height of the proximal wall of the upper cage is greater than the height of the anterior wall of the upper cage.

In some embodiments, the proximal wall of the upper cage has an upper portion 35 having a upper through-hole 37 located above the anterior wall and adapted for receiving a bone screw.

In some embodiments, the proximal wall of the lower cage has a height H1-p, the anterior wall of the lower cage has a height H1-a, and wherein the height of the proximal wall of the lower cage is greater than the height of the anterior wall of the lower cage.

In some embodiments, the proximal wall of the lower cage has a lower portion 39 having a lower through-hole 41 located beneath the anterior wall and adapted for receiving a bone screw.

In some locking plate embodiments, the proximal wall of the upper cage has a lower portion 43 having an lower through-hole 45 located beneath the anterior wall of the upper cage and adapted for receiving a screw. Likewise, the proximal wall of the lower cage has an upper portion 47 having an upper through-hole 49 located above the anterior wall of the lower cage and adapted for receiving a bone fastener such as a screw. The device further comprises:
c) a locking plate 51 having a first and second throughholes 53, and
d) first and second bone fasteners (such as screws) 55,
wherein the locking plate is fixed to the proximal wall of the upper and lower cages by passing the first bone fastener through the first throughhole of the locking plate and into the lower throughhole of the upper cage, and by passing the second bone fastener through the second throughhole of the locking plate and into the upper throughhole of the lower cage.

In some embodiments that promote fusion, the upper cage further comprises a lower surface 13 and a throughole 59 running from the upper surface to the lower surface. In some embodiments that promote fusion, the lower cage further comprises an upper surface and a throughole running from the upper surface to the lower surface. Likewise,
the anterior wall further comprises a throughole 61 running therethrough. These throughholes are of a size adapted to promote fusion

In some embodiments, the distal end wall of each of the upper and lower cages has a taper 63 for ease of insertion.

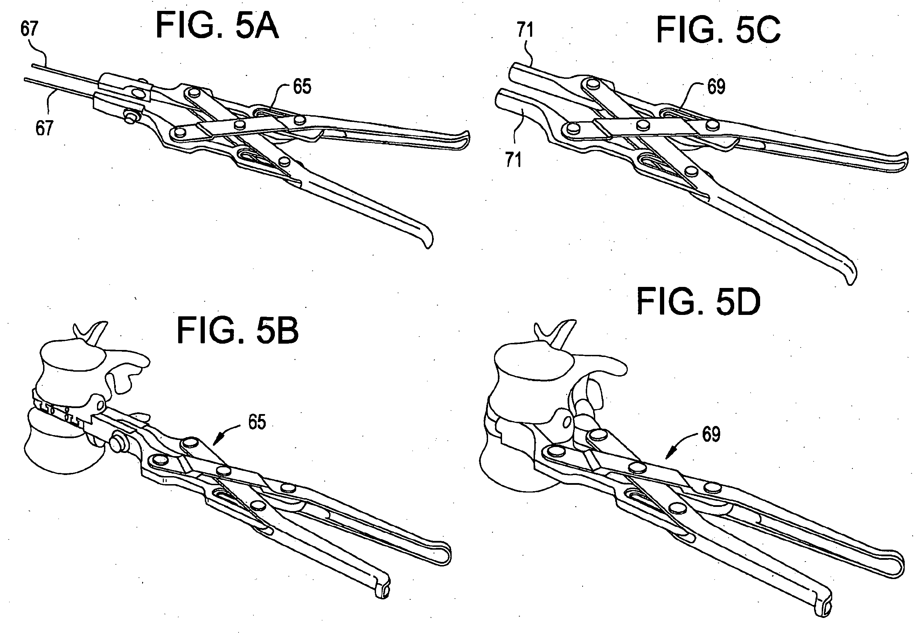

In the first embodiment, and now referring to FIGS. 5a-7b, the alignment means is compression-activated. This preferred embodiment uses a compression instrument to bring the anterior and posterior walls of the inferior and superior cages into alignment, and thereby correct spondylolisthesis.

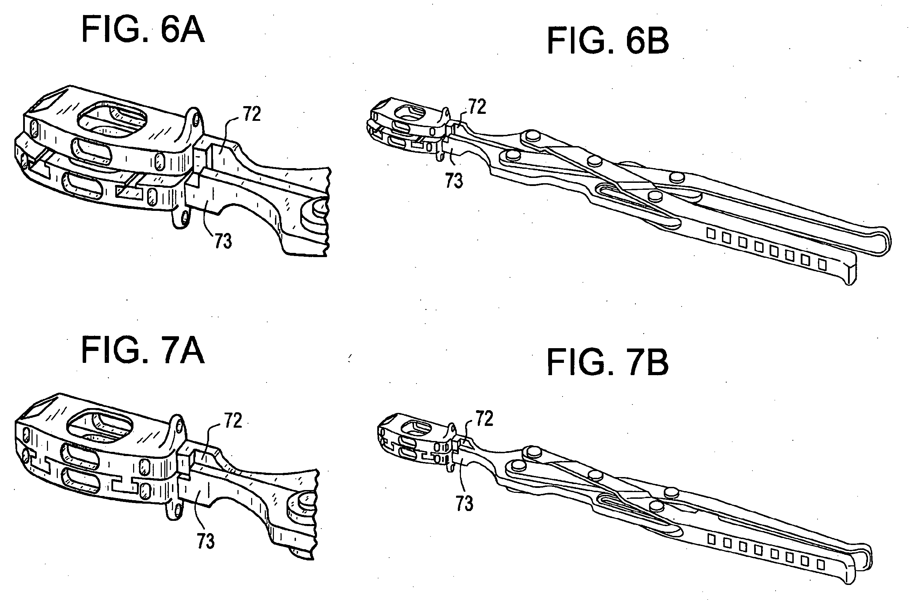

FIGS 5a-b disclose a compression-directed inserter 65 having distal pins 67, and the insertion of a cage of the present invention with this inserter. FIGS 5c-d disclose a compression-directed inserter 69 having distal blades 71, and the insertion of a cage of the present invention with this inserter. FIGS. 6a-7b disclose how a compression-directed inserter having nested blades 72,73 aligns the cages of the present invention when activated.

Now referring to FIG. 5a-d, the compressor instrument may have distal extensions such as blades 71 (FIG. 5c) or pins 67 (FIG. 5a) that act to align the anterior and posterior walls of the cages via compression. The blades of FIG. 5c simply push the anterior and posterior walls of the cages towards each other, thereby removing any overlap and bringing the anterior and posterior walls of these cages into vertical alignment. FIGS. 6a-7b disclose the nesting details of the distal portions of the pinned compressor instrument that allow the instrument's distal pins (not shown) to become aligned.

Therefore, there is provided a method for correcting spondylolisthesis in a patient, comprising the steps of:
a) selecting a fusion device comprising an upper cage and a lower cage,
b) fixing the upper cage to an upper vertebral body of the patient and the lower cage to a lower vertebral body of the patient,
c) moving the upper cage relative to the lower cage to correct the spondylolisthesis.
Preferably, the method further comprises the step of: d) locking the upper cage to the lower cage.
In some embodiments, the locking step is accomplished by a locking plate. In some embodiments, the moving step is accomplished with a compression-directed inserter.

In some embodiments, the moving step is accomplished with a rotary spreader.

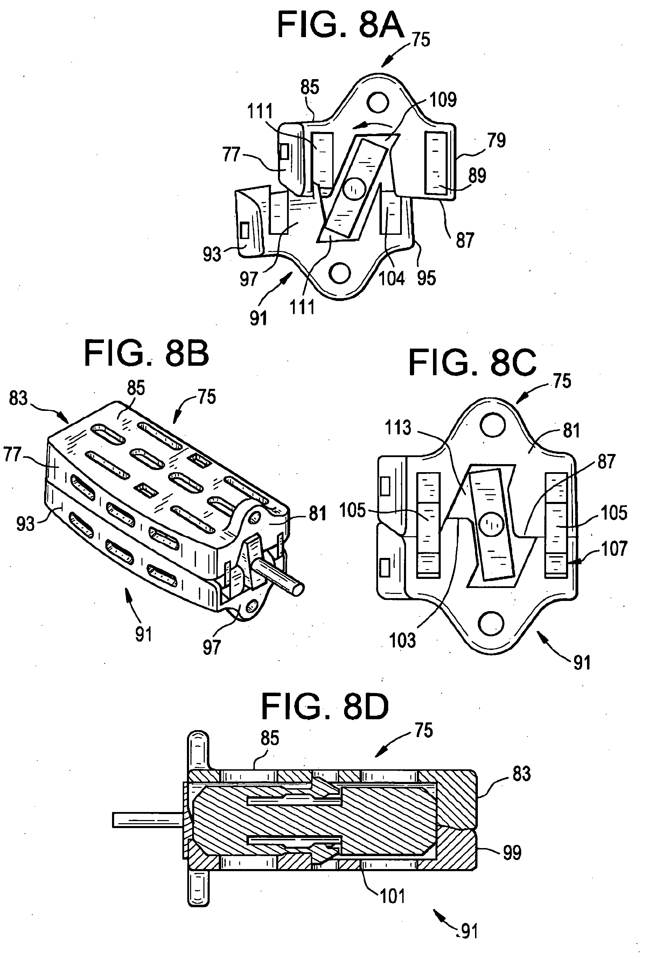

In a second embodiment, and now referring to FIG. 8a-8d, the alignment means is rotary spreader-activated. FIGS. 8a-8d disclose how the cages of one embodiment of the present invention are aligned by a rotary spreader, and are locked by a particular locking plate. A modified spreader or shaver can be inserted into a space formed in the proximal end wall of the unaligned device. Rotating the spreader causes relative anterior-posterior movement of the upper cage vis-a-vis the lower cage to enable alignment of the cages and thereby intraoperative adjustment of a spondylosed functional spinal unit (FSU).

Now referring to FIG. 8b, optional locking plates can be employed to fix the device after the spondylolisthesis has been corrected. These plates are preferably inserted into bilateral, aligned, longitudinal recesses that extend across the interface of the aligned cages to provide inter-cage locking. In some embodiments, these plates are locked into place via a snap-lock mechanism, as shown in FIG. 8d.

In some embodiments, and now referring to FIG. 8a, the upper surface of the lower cage and the lower surface of the upper cage may be configured in matching domes in order to mimic the natural relative arced movement of adjacent vertebral bodies.

Now referring to FIGS. 8a-8d, there is provided an intervertebral fusion device for correcting spondylolisthesis in a patient, comprising:
a) an upper cage 75 having an anterior wall 77, a posterior wall 79, and a proximal wall 81 and a distal wall 83 connecting the anterior and posterior walls, and an upper surface 85 for contacting an upper vertebral body and a lower surface 87 having a first groove 89 therein,
b) a lower cage 91 having an anterior wall 93, a posterior wall 95, and a proximal wall 97 and a distal wall 99 connecting the anterior and posterior walls, and a lower surface 101 for contacting a lower vertebral body and an upper surface 103 having a second groove 104 therein, and
c) a pair of locking plates 105,
wherein the lower surface of the upper cage contacts the upper surface of the lower cage so that the first and second grooves form a first throughhole 107 running from the proximal wall to about the distal wall,
wherein the locking plate is disposed in the first throughole.

In some embodiments, the first groove is present upon the lower surface of the anterior wall of the upper cage, and the second groove is present upon the upper surface of the anterior wall of the lower cage. In other embodiments, the first groove is present upon the lower surface of the posterior wall of the upper cage, and the second groove is present upon the upper surface of the posterior wall of the lower cage.

In some embodiments, the device of the second embodiment further comprises a third groove 109 present upon the lower surface of the upper cage between the anterior and posterior walls, and a fourth groove 111 present upon the upper surface of the lower cage between the anterior and posterior walls, and wherein the lower surface of the upper cage contacts the upper surface of the lower plate so that the third and fourth grooves form a second throughhole 113 running from the proximal wall to about the distal wall, the second throughhole adapted for insertion of a spreader therein.

In a third embodiment, and now referring to FIGS. 9a-9g, the attachment means is linkage activated. FIGS. 9a-9g disclose various views of a dual linkage embodiment of the present invention Single- or double-linkage can be used to correct spondylolisthesis by moving this cage from a pre-activated (FIG. 9a) to a post-activated state. (FIG. 9b) In some linkage embodiments, the anterior and posterior walls of the cages also function as linkage bars, providing for pivoting connection with both an upper wall component and a lower wall component to allow for relative anterior-posterior movement of the upper wall vis-à-vis the lower wall and thereby spondylolisthesis correction.

In some embodiments, and now referring to FIGS. 9c-9d, the upper portion of the upper wall and the lower portion of the lower wall extend outwardly, and transverse holes in these portions provide a means to fix the upper and lower walls to the respective lateral walls of the vertebral bodies.

In some embodiments, and now referring to FIG. 9d, the upper wall and the lower wall have transverse throughholes that extend into a chamber formed in the interior of the device. These throughholes and this chamber facilitate the fusion of the opposing vertebral bodies through the device. Also referring to FIG. 9d, the anterior and posterior walls may likewise have transverse throughholes that extend into a chamber formed in the interior of the device, and thereby facilitate the fusion of the opposing vertebral bodies through the device.

Now referring to FIG. 9e-9g, optional locking plates can be employed to fix the device following spondylolisthesis correction. These plates are preferably inserted into bilateral, aligned, longitudinal recesses that extend from the upper wall to the lower wall to provide inter-cage locking. In some embodiments, these plates are locked into place via a snap-lock mechanism, as shown in FIG. 9e.

Now referring to FIGS. 9a-9g, there is provided an intervertebral fusion device for correcting spondylolisthesis in a patient, comprising:
a) an upper cage 115 having an anterior wall 117, a posterior wall 119, and a proximal wall 121 and a distal wall 123 connecting the anterior and posterior walls, and an upper surface 125 for contacting an upper vertebral body and a lower surface 127,
b) a lower cage 131 having an anterior wall 133, a posterior wall 135, and a proximal wall 137 and a distal wall 139 connecting the anterior and posterior walls, and a lower surface 141 for contacting a lower vertebral body and an upper surface 143,
wherein the anterior wall is connected to the proximal wall by a first dual linkage 145 and to the distal wall by a second dual linkage (not shown),
wherein the posterior wall is connected to the proximal wall by a third dual linkage 149 and to the distal wall by a fourth dual linkage (not shown),
wherein the linkages allow the upper plate to pivot relative to the lower plate in the plane of the proximal wall.

In some aspects of this third embodiment, the proximal wall of the upper cage has a height, the anterior wall of the upper cage has a height, and wherein the height of the proximal wall of the upper cage is greater than the height of the anterior wall of the upper cage. In some embodiments thereof, the proximal wall of the upper cage has an upper portion 153 having a upper through-hole 155 located above the anterior wall and adapted for receiving a bone screw.

In other aspects of this third embodiment, the proximal wall of the lower cage has a height, the anterior wall of the lower cage has a height, and wherein the height of the proximal wall of the lower cage is greater than the height of the anterior wall of the lower cage. In some embodiments thereof, the proximal wall of the lower cage has a lower portion 157 having a lower through-hole 159 located beneath the anterior wall and adapted for receiving a bone screw.

In some embodiments, the upper cage has a throughole 161 running from the upper surface to the lower surface. This throughhole is adapted for promoting fusion

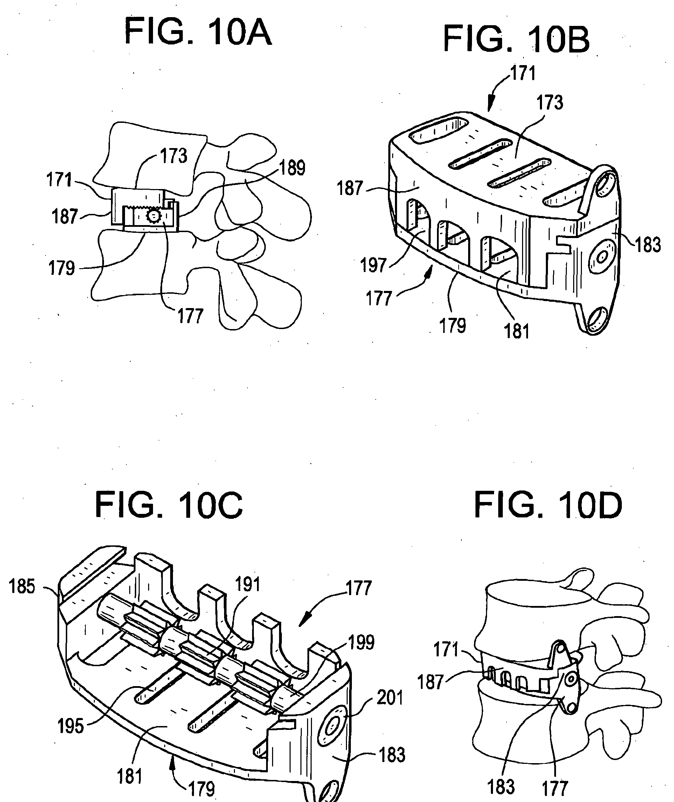

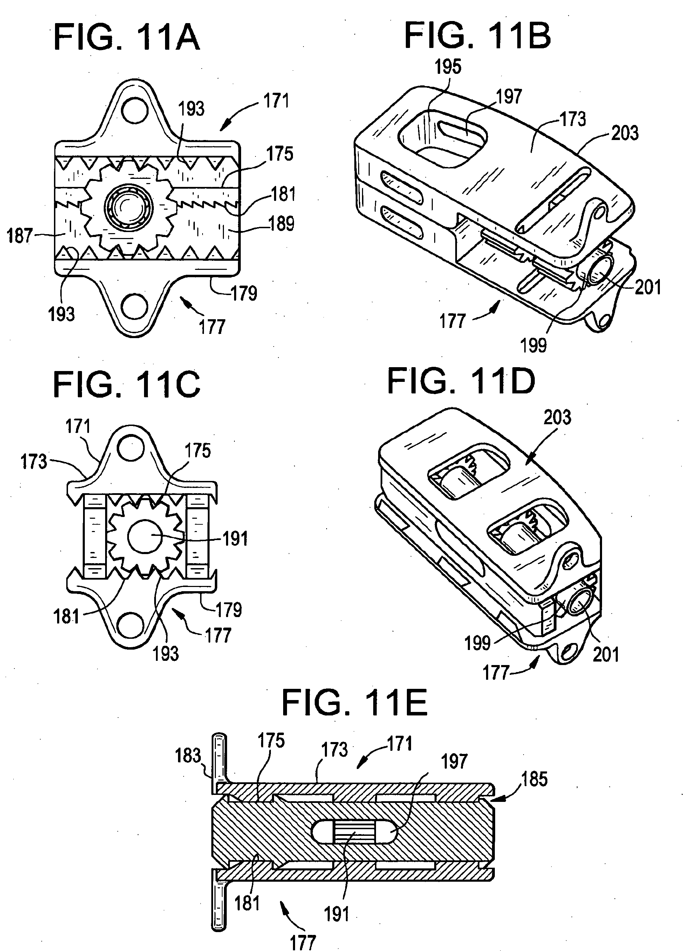

In a fourth embodiment, the alignment means includes a rack-and-pinion. A pinion located between the upper and lower walls and extending laterally can be rotated to move racks extending in the anterior-posterior direction and thereby reduce spondylolisthesis. FIGS. 10a-d disclose full and partial views of a rack-and-pinion embodiment of the present invention, some of which are inserted into a disc space. FIGS 11a-11e disclose various views of the rack-and-pinion embodiment of the present invention.

Now referring to FIGS. 10a-11e, there is provided an intervertebral fusion device for correcting spondylolisthesis in a patient, comprising:
a) an upper wall 171 having an upper surface 173 adapted for contacting an upper vertebral body and an inner surface 175,
b) a lower wall 177 having a lower surface 179 adapted for contacting a lower vertebral body and an inner surface 181,
c) proximal 183 and distal 185 walls extending between the upper and lower walls,
d) anterior 187 and posterior 189 walls extending between the upper and lower walls,
e) a rack-and-pinion mechanism located between the inner surfaces of the upper and lower walls
wherein the pinion 191 extends substantially from the distal wall to the proximal wall, wherein the rack 193 extends substantially from the anterior wall to the posterior wall,
so that rotation of the pinion effects relative movement of the upper and lower walls in the anterior-posterior direction.

In some aspects of the fourth embodiment, the length of the device is at least three times the height of the device.

In some embodiments, the upper and lower walls each have at least one hole 195 therethrough to facilitate fusion through the device. In others, the anterior and posterior walls each have at least one hole 197 therethrough to facilitate fusion through the device.

In some embodiments, the rack extends from the inner surface of the upper wall. In others, the rack extends from the inner surface of the lower wall.

In some embodiments, the pinion comprises a proximal end 199 having a feature 201 for receiving a rotary tool.

In some embodiments, at least one of the anterior and posterior walls is integral with at least one of the upper and lower walls.

In some embodiments, at least one of the anterior and posterior walls is removable.

The embodiments of the present invention may optionally a securement plate that attaches to both the device of the present invention and the vertebral bodies. This securement plate secures the position of the device and provides supplemental stabilization.

In general, the devices of the present invention are suited for substantially lateral insertion into the disc space. In some embodiments, the cages are inserted through a more anterolateral insertion angle.

Now referring to FIGS 4a and 4c, the length L of the device is characterized as the distance from the distal wall to the proximal wall. The width W of the device is characterized as the distance from the anterior wall to the posterior wall. The height H of the device is characterized as the distance from the lower surface to the upper surface, excludes the upper and lower portions that extend past the anterior wall, and generally corresponds to the height of the disc space. In general, the length of the lateral devices of the present invention are typically at least twice and often three times the width of the device. In general, the length of the lateral devices of the present invention are typically at least twice and often three times the height of the device. Typically, the width of the device is greater than the height of the device.

In some embodiments, as in FIG. 11b, the anterior wall of the cage may have a convex curve 203 to mimic the convex shape of the anterior portion of the disc space

The lateral spondylolisthesis reduction fusion devices of the present invention may be produced from a single material or from multiple materials. These materials include metallics (such as Ti, Ti alloys such as nitinol, stainless steel, and cobalt-chrome), polymeric materials (including PEEK, PEAK, polypropylene, polyethylene terephthalate (PET), UHMWPE), biologic materials (including allograft, hydroxyapatite, TCP and CaPO₄), and ceramic materials including silicon nitrides, and zirconia-containing ceramics. The plate, fasteners, or locking mechanisms can be produced from metallics or polymers for enhance durability.

Additionally, modified versions of this concept can be designed to correct spondylolisthesis with superior and inferior cages that are inserted from the anterior, anterior-lateral or posterior approaches.

The cages of the present invention are preferably inserted either from a right lateral or left lateral approach.

Following standard access and disc preparation procedures, the superior and inferior cages are inserted and affixed to the opposed vertebral bodies with screws or bone fasteners. Spondylolisthesis correction is then performed with the disclosed compressor or with a rotary tool. Optionally, locking members are then applied to the superior and inferior cages to fix the orientation of the segments.

Also there is provided a method of implanting an intervertebral device between opposed vertebral bodies, comprising the steps of:
i) selecting an intervertebral device comprising:
   a. an upper half component having an anterior wall, a posterior wall, and two side walls connecting the anterior wall and posterior wall;
   b. a lower half component having an anterior wall, a posterior wall, and two side walls connecting the anterior wall and posterior wall;
ii) inserting the device between opposed vertebral bodies, whereby the anterior walls are not aligned,
iii) moving (preferably by pivoting) one of the components with respect to the other component so that the anterior walls are substantially aligned, and
iv) fixing the device to the opposed vertebral bodies. ,

In some embodiments of the present invention, the fusion device is angled to provide either lordosis or kyphosis. In embodiments in which lordosis is desired, the height of the anterior wall exceeds the height of the posterior wall. An example of such a lordotic implant is shown in FIG. 4c. In embodiments in which kyphosis is desired, the height of the anterior wall is less than the height of the posterior wall.

It is believed by the present inventors that the devices disclosed herein appear to be the first intervertebral devices having a flange that connects to a side of a vertebral body. Therefore, there is provided a method of inserting a fusion device between opposed vertebral bodies, comprising the steps of :
a) selecting an intervertebral device having an anterior wall, a posterior wall and a pair of side walls connecting the anterior and posterior walls, wherein at least one of the side walls has a flange axially extending beyond the anterior wall and the posterior wall, wherein the flange has a throughhole,
b) inserting the device between the opposed vertebral bodies, and
c) inserting a fixation device through the throughhole to fix the device to a side of one of the opposed vertebral bodies.

Although the cages of the present invention are disclosed as having flanges that extend beyond the disc space for attachment to the sides of the opposed vertebral bodies, it is also contemplated that the cages of the present invention may be attached to the opposed vertebral bodies via zero profile throughholes. These zero profile throughholes are provided both a) at the upper edge of the proximal side wall of the upper half component and b) at the lower edge of the proximal side wall of the lower half component.

Therefore, in accordance with the present invention, there is provided an intervertebral fusion device for correcting spondylolisthesis in a patient, comprising:
a) an upper cage having an anterior wall, a posterior wall, and a proximal wall and a distal wall connecting the anterior and posterior walls, and a throughole present at the upper edge of the proximal wall for receiving a bone fixation device,
b) a lower cage having an anterior wall, a posterior wall, and a proximal wall and a distal wall connecting the anterior and posterior walls, and a lower surface for contacting a lower vertebral body and an upper surface, and a throughole present at the lower edge of the proximal wall for receiving a bone fixation device,
wherein the lower surface of the upper cage slidingly mates with upper surface of the lower cage.

Although the above description discloses how to make and use implantable devices to correct spondylolisthesis, it is within the scope of the invention to use these devices as instruments to correct retrolisthesis as well. Therefore, there is provided a method for correcting spondylolisthesis in a patient, comprising the steps of:
a) selecting an instrument comprising an upper cage and a lower cage, wherein each cage is attached to a handle
b) attaching the upper cage to an upper vertebral body of the patient and the lower cage to a lower vertebral body of the patient (preferably with caspar pins),
c) moving the upper cage relative to the lower cage to correct the spondylolisthesis (preferably with a distractor that engages the caspar pins), and
d) removing the instrument from the patient..

Although the above description discloses how to make and use devices in the context of correcting spondylolisthesis, it is within the scope of the invention to use similar devices to correct retrolisthesis as well.

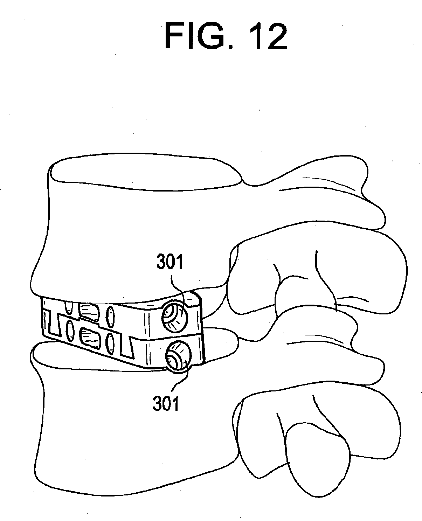

FIG. 12 discloses a lateral cage of the present invention with oblique screwholes 301.

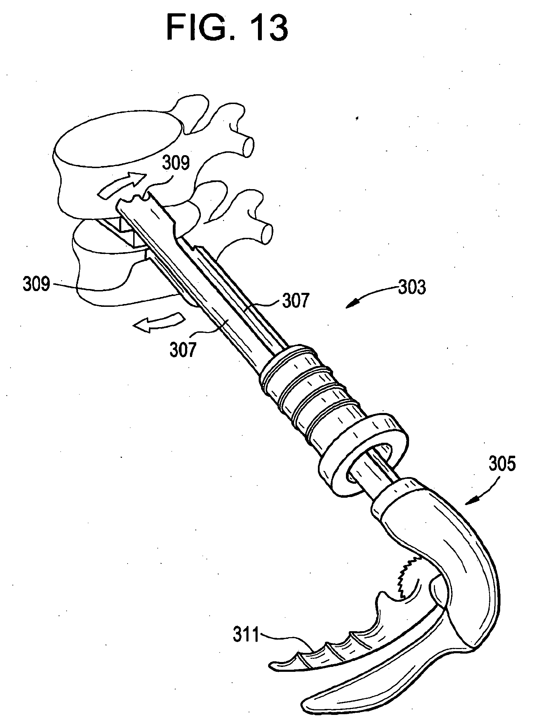

FIG. 13 discloses an inserter 303. Figure 13 is a top view of a second type of spondylolisthesis reduction tool that comprises a proximal handle portion 305 and two vertebral body engaging beams 307. On the distal end of the beams are bone engaging features 309for the respective superior and inferior vertebral bodies. At the proximal end of the beams the inferior beam is fixed within the handle and the superior is attached with a pivot so that its distal end can move posterior and anterior with respect to the lower. Conversely, the lower beam could also be affixed in a pivoting fashion so that both beams move as in a scissor fashion. This intended motion corresponds to a posterior transverse plane motion of the superior vertebral body in order to reduce the spondylolisthesis. By slightly rotating the handle or tilting the tool prior to engagement, a saggital plane component is introduced to the reduction motion (it may be beneficial to increase the height of one body over the other as you move that body posteriorly).

The cross section of the beams are sufficiently wide in the anterior-posterior direction making them resistant to bending in the transverse plane. The mechanism within the handle is to pivot the beams. This can be done with a ratchet and pawl linkage which moves the top beam one click with each squeeze, or a sliding collar that advances distally along the beams to bring them in line with each other, or a wedge/roller that advances along the edge of the superior beam or a post and angled slot mechanism that aligns the two beams, or with a geared scissor mechanism such that the full motion of the handle corresponds to a small angular change of the beams. The controlled motion of the beams relative to each other is advantageous as the operating surgeon generally has a pre-determined amount of reduction in mind for the surgery. This amount can be determined via radiograph or inter-operatively. For example if a total of 6 mm of reduction is desired, the handle can be ratcheted 1 mm at a time until the value of 6mm is reached.

Therefore, there is provided a spondylolisthesis reduction tool comprising:
a) a proximal handle portion, and
b) first and second vertebral body-engaging beams having a longitudinal axis, a proximal end portion and a distal end portion, the distal end portion of each beam forming bone engaging features,
wherein the proximal end portion of the first beams is fixedly attached to the handle portion, and
wherein the proximal end portion of the second beam is pivotally attached to the handle portion
so that the second beam can move transversely with respect to the longitudinal axis of the first beam.
Preferably, the handle portion comprises a trigger 311 adapted to pivotally move the second beam.

## Claims

1. An intervertebral fusion device for correcting spondylolisthesis in a patient, comprising:
a) an upper cage (1) having an anterior wall (3), a posterior wall (5), and a proximal wall (7) and a distal wall (9) connecting the anterior and posterior walls, an upper surface (11) for contacting an upper vertebral body and a lower surface (13), wherein the proximal wall has an upper portion (35) having an upper through-hole (37) located above the anterior wall and adapted for receiving a fastener,
b) a lower cage (15) having an anterior wall (17), a posterior wall (19), and a proximal wall (21) and a distal wall (23) connecting the anterior and posterior walls, a lower surface (15) for contacting a lower vertebral body and an upper surface (27), wherein the proximal wall has a lower portion (39) having a lower through-hole (41) located beneath the anterior wall and adapted for receiving a fastener,
wherein the proximal wall of the upper cage has a lower portion (43) having a lower through-hole (45) adapted for receiving a bone fastener (55), and the proximal wall of the lower cage has an upper portion (47) having an upper through-hole (49) adapted for receiving a bone fastener (55),
c) bone fasteners (55) disposed in the upper and lower through-holes of the upper and lower cages,
wherein the lower surface of the upper cage slidingly mates with the upper surface of the lower cage to allow linear anterior-posterior relative movement of the cages,
wherein the lower surface of the upper cage and the upper surface of the lower cage have matching ratchet teeth (33).

2. The device of claim 1 wherein combined heights (Hᵤ₋ₐ; Hₗ₋ₐ) of the anterior walls exceed the combined heights of the posterior walls.

3. The device of claim 1 wherein the proximal wall of the upper cage has a height (Hᵤ₋ₚ), the anterior wall of the upper cage has a height (Hᵤ₋ₐ), and wherein the height of the proximal wall of the upper cage is greater than the height of the anterior wall of the upper cage.

4. The device of claim 1 wherein the proximal wall of the lower cage has a height (Hₗ₋ₚ), the anterior wall of the lower cage has a height (Hₗ₋ₐ), and wherein the height of the proximal wall of the lower cage is greater than the height of the anterior wall of the lower cage.

5. The device of claim 1 further comprising a locking plate (51) having first and second throughholes (53), wherein the locking plate is fixed to the proximal wall of the upper and lower cages by passing a first one of the bone fasteners (55) through the first throughhole of the locking plate and into the lower throughhole (45) of the upper cage, and by passing a second one of the bone fasteners (55) through the second throughole of the locking plate and into the upper throughhole (49) of the lower cage.

6. The device of claim 1 wherein the upper cage further comprises a throughole (59) running from the upper surface to the lower surface.

7. The device of claim 1 wherein the anterior wall further comprises a throughole (61) running therethrough.

8. The device of claim 1 wherein the distal end wall of each of the upper and lower cages has a taper for ease of insertion.

9. An intervertebral fusion device as claimed in claim 1, in which the upper through-hole of the upper cage is present at an upper edge of the proximal wall, and the lower through-hole of the lower cage is present at a lower edge of the proximal wall.

## Patentansprüche

1. Zwischenwirbelfusionsvorrichtung zum Korrigieren einer Spondylolisthesis bei einem Patienten, umfassend:
a) einen oberen Korb (1) mit einer vorderen Wand (3), einer hinteren Wand (5) sowie einer proximalen Wand (7) und einer distalen Wand (9), welche die vordere und die hintere Wand verbinden, einer oberen Fläche (11) zum Kontaktieren eines oberen Wirbelkörpers und einer unteren Fläche (13), wobei die proximale Wand einen oberen Abschnitt (35) hat, welcher eine obere Durchgangsbohrung (37) aufweist, die oberhalb der vorderen Wand gelegen und zum Aufnehmen eines Befestigungsmittels geeignet ist,
b) einen unteren Korb (15) mit einer vorderen Wand (17), einer hinteren Wand (19) sowie einer proximalen Wand (21) und einer distalen Wand (23), welche die vordere und die hintere Wand verbinden, einer unteren Fläche (15) zum Kontaktieren eines unteren Wirbelkörpers und einer oberen Fläche (27), wobei die proximale Wand einen unteren Abschnitt (39) hat, welcher eine untere Durchgangsbohrung (41) aufweist, die unterhalb der vorderen Wand gelegen und zum Aufnehmen eines Befestigungsmittels geeignet ist,
wobei die proximale Wand des oberen Korbs einen unteren Abschnitt (43) hat, welcher eine zum Aufnehmen eines Knochen-Befestigungsmittels (55) geeignete untere Durchgangsbohrung (45) hat, und die proximale Wand des unteren Korbs einen oberen Abschnitt (47) hat, welcher eine zum Aufnehmen eines Knochen-Befestigungsmittels (55) geeignete obere Durchgangsbohrung (49) hat,
c) Knochen-Befestigungsmittel (55), welche in der oberen und der unteren Durchgangsbohrung des oberen und des unteren Korbs angeordnet sind,
wobei die untere Fläche des oberen Korbs gleitend mit der oberen Fläche des unteren Korbs in Kontakt steht, um eine lineare anterior-posteriore Relativbewegung der Körbe zu ermöglichen,
wobei die untere Fläche des oberen Korbs und die obere Fläche des unteren Korbs zusammen passende Sperrzähne (33) haben.

2. Vorrichtung nach Anspruch 1, wobei die kombinierten Höhen (Hᵤ₋ₐ; Hₗ₋ₐ) der vorderen Wände größer als die kombinierten Höhen der hinteren Wände sind.

3. Vorrichtung nach Anspruch 1, wobei die proximale Wand des oberen Korbs eine Höhe (Hᵤ₋ₚ) hat, die vordere Wand des oberen Korbs eine Höhe (Hᵤ₋ₐ) hat, und wobei die Höhe der proximalen Wand des oberen Korbs größer als die Höhe der vorderen Wand des oberen Korbs ist.

4. Vorrichtung nach Anspruch 1, wobei die proximale Wand des unteren Korbs eine Höhe (Hₗ₋ₚ) hat, die vordere Wand des unteren Korbs eine Höhe (Hₗ₋ₐ) hat, und wobei die Höhe der proximalen Wand des unteren Korbs größer als die Höhe der vorderen Wand des unteren Korbs ist.

5. Vorrichtung nach Anspruch 1, ferner umfassend eine Sicherungsplatte (51) mit einer ersten und einer zweiten Durchgangsbohrung (53), wobei die Sicherungsplatte durch Hindurchführen eines ersten der Knochen-Befestigungsmittel (55) durch die erste Durchgangsbohrung der Sicherungsplatte und in die untere Durchgangsbohrung (45) des oberen Korbs und durch Hindurchführen eines zweiten der Knochen-Befestigungsmittel (55) durch die zweite Durchgangsbohrung des Sicherungsplatte und in die obere Durchgangsbohrung (49) des unteren Korbs an der proximalen Wand des oberen und des unteren Korbs befestigt ist.

6. Vorrichtung nach Anspruch 1, wobei der obere Korb ferner eine Durchgangsbohrung (59) hat, welche von der oberen Fläche zu der unteren Fläche verläuft.

7. Vorrichtung nach Anspruch 1, wobei die vordere Wand ferner eine durch sie hindurch verlaufende Durchgangsbohrung (61) hat.

8. Vorrichtung nach Anspruch 1, wobei die distale Endwand des oberen und des unteren Korbs jeweils eine Abschrägung zum leichten Einführen hat.

9. Zwischenwirbelfusionsvorrichtung nach Anspruch 1, bei der die obere Durchgangsbohrung des oberen Korbs an einer oberen Kante der proximalen Wand vorhanden ist und die untere Durchgangsbohrung des unteren Korbs an einer unteren Kante der proximalen Wand vorhanden ist.

## Revendications

1. Dispositif de fusion intervertébrale pour corriger un spondylolisthésis chez un patient, comprenant :
a) une cage supérieure (1) ayant une paroi antérieure (3), une paroi postérieure (5) ainsi qu'une paroi proximale (7) et une paroi distale (9) connectant les parois antérieure et inférieure, une surface supérieure (11) pour entrer en contact avec un corps vertébral supérieur et une surface inférieure (13), dans lequel la paroi proximale a une portion supérieure (35) ayant un trou débouchant supérieur (37) situé au-dessus de la paroi antérieure et adapté pour recevoir un élément de fixation,
b) une cage inférieure (15) ayant une paroi antérieure (17), une paroi postérieure (19) ainsi qu'une paroi proximale (21) et une paroi distale (23) connectant les parois antérieure et inférieure, une surface inférieure (15) pour entrer en contact avec un corps vertébral inférieur et une surface supérieure (27), dans lequel la paroi proximale a une portion inférieure (39) ayant un trou débouchant inférieur (41) situé au-dessous de la paroi antérieure et adapté pour recevoir un élément de fixation,
dans lequel la paroi proximale de la cage supérieure a une portion inférieure (43) ayant un trou débouchant inférieur (45) adapté pour recevoir un élément de fixation d'os (55), et la paroi proximale de la cage inférieure a une portion supérieure (47) ayant un trou débouchant supérieur (49) adapté pour recevoir un élément de fixation d'os (55),
c) des éléments de fixation d'os (55) disposés dans les trous débouchants supérieur et inférieur des cages supérieure et inférieure,
dans lequel la surface inférieure de la cage supérieure s'accouple en coulissement avec la surface supérieure de la cage inférieure pour permettre un déplacement relatif antérieur-postérieur linéaire des cages,
dans lequel la surface inférieure de la cage supérieure et la surface supérieure de la cage inférieure comportent des dents d'encliquetage correspondantes (33).

2. Dispositif selon la revendication 1 dans lequel les hauteurs combinées (Hᵤ₋ₐ, Hₗ₋ₐ) des parois antérieures dépassent les hauteurs combinées des parois postérieures.

3. Dispositif selon la revendication 1 dans lequel la paroi proximale de la cage supérieure a une hauteur (Hᵤ₋ₚ), la paroi antérieure de la cage supérieure a une hauteur (Hᵤ₋ₐ), et dans lequel la hauteur de la paroi proximale de la cage supérieure est supérieure à la hauteur de la paroi antérieure de la cage supérieure.

4. Dispositif selon la revendication 1 dans lequel la paroi proximale de la cage inférieure a une hauteur (Hₗ₋ₚ), la paroi antérieure de la cage inférieure a une hauteur (H₁₋ₐ), et dans lequel la hauteur de la paroi proximale de la cage inférieure est supérieure à la hauteur de la paroi antérieure de la cage inférieure.

5. Dispositif selon la revendication 1 comprenant en outre une plaque de verrouillage (51) ayant des premier et deuxième trous débouchants (53), dans lequel la plaque de verrouillage est fixée à la paroi proximale des cages supérieure et inférieure en faisant passer un premier des éléments de fixation d'os (55) dans le premier trou débouchant de la plaque de verrouillage et dans le trou débouchant inférieur (45) de la cage supérieure, et en faisant passer un deuxième des éléments de fixation d'os (55) dans le deuxième trou débouchant de la plaque de verrouillage et dans le trou débouchant supérieur (49) de la cage inférieure.

6. Dispositif selon la revendication 1 dans lequel la cage supérieure comprend en outre un trou débouchant (59) s'étendant de la surface supérieure à la surface inférieure.

7. Dispositif selon la revendication 1 dans lequel la paroi antérieure comprend en outre un trou débouchant (61) s'étendant à travers celle-ci.

8. Dispositif selon la revendication 1 dans lequel la paroi d'extrémité distale de chacune des cages supérieure et inférieure présente un rétrécissement pour faciliter l'insertion.

9. Dispositif de fusion intervertébrale selon la revendication 1, dans lequel le trou débouchant supérieur de la cage supérieure est présent au niveau d'un bord supérieur de la paroi proximale, et le trou débouchant inférieur de la cage inférieur est présent au niveau d'un bord inférieur de la paroi proximale.
